Europäisches Patentamt

European Patent Office

Office européen des brevets

(19)

(11) **EP 1 496 119 A1**

(12) **EUROPEAN PATENT APPLICATION**
published in accordance with Art. 158(3) EPC

(43) Date of publication:
**12.01.2005 Bulletin 2005/02**

(21) Application number: **03745211.7**

(22) Date of filing: **01.04.2003**

(51) Int Cl.⁷: **C12N 15/09**, C12N 5/10,
C12Q 1/02, G01N 33/50

(86) International application number:
**PCT/JP2003/004180**

(87) International publication number:
**WO 2003/083112 (09.10.2003 Gazette 2003/41)**

(84) Designated Contracting States:
**AT BE BG CH CY CZ DE DK EE ES FI FR GB GR
HU IE IT LI LU MC NL PT RO SE SI SK TR**

(30) Priority: **01.04.2002 JP 2002099323
01.04.2002 JP 2002099339
01.04.2002 JP 2002099350**

(71) Applicants:
• **Japan Science and Technology Agency
Kawaguchi-shi, Saitama 332-0012 (JP)**
• **Japan as represented by Director General of
National Rehabilitation Center for Persons with
Disabilities
Tokorozawa-shi, Saitama 359-8555 (JP)**

(72) Inventors:
• **KATO, Seishi
Sagamihara-shi, Kanagawa 229-0014 (JP)**
• **FUJIMORI, Fumie
Nishitokyo-shi, Tokyo 202-0015 (JP)**

(74) Representative: **Webber, Philip Michael
Frank B. Dehn & Co.,
179 Queen Victoria Street
London EC4V 4EL (GB)**

(54) **CELL POPULATION PROVIDED WITH IDENTIFICATION CODES AND METHOD OF
SCREENING CELL POPULATION**

(57)    The invention of this application provides a cell population with identification codes, which is a population of cells that can be distinguished from one another based on a difference in luminescent signals emitted by luminescent substances, wherein the difference in the luminescent signals is caused by either or both (a) 2 or more different luminescent properties, and (b) 2 or more different luminescent sites. This cell population with identification codes can be conveniently prepared without a resort to a special apparatus and can be applied to either a solid phase system or a liquid phase system in mass screening, and moreover, can be used for a screening for various properties of cells such as the expression of a target protein.

Fig. 1

## Description

### Technical Field

The invention of this application relates to a cell population with identification codes and a method of screening this cell population. More particularly, the invention of this application relates to a cell population in which respective cells can be distinguished from one another based on a difference in luminescent signals as the identification code, and which is useful for a gene search by expression cloning, detection of protein-protein interaction, or the like, and an invention for using this cell population.

### Background Art

In genome projects, a number of new genes have been discovered. In order to investigate the functions of the proteins encoded by these new genes or to develop a new drug with the use of these proteins, a substance binding to these proteins (target proteins) needs to be found. Therefore, various assay methods for this purpose have been developed (E. M. Phizicky and S. Fields, Microbiol. Rev. 59: 94-123, 1995; A. R. Mendelsohn and R. Brent, Science 284: 1948-1950, 1999). The most common method is a method of immobilizing plural target proteins on a support, allowing labeled probes to act on them and investigating whether or not the probes will bind to them. Conventionally, the Western blotting method and the ELISA method, in which immobilization of target proteins is easy, have been widely used; however, there are the following problems.

(1) A large amount of target proteins needs to be prepared.
(2) It requires time and work to isolate and purify target proteins.
(3) Target proteins may be decomposed or denatured in the process of isolation and purification or immobilization in some cases.
(4) A large amount of probes is needed for screening.

In order to solve the problems of (1) and (4) among them, the protein microarray method was developed recently. More specifically, it is a method of using a protein chip in which target proteins are immobilized in a minute area on a slide glass at a high density in a lattice pattern (MacBeath & Schreiber, Science 289: 1760-1763, 2000).

However, even with this protein chip, the problems of (2) and (3) are left unsolved. Therefore, in order to omit the isolation and purification process of target proteins, a method of immobilizing expression vectors of target proteins on spots in a lattice pattern on a slide glass, culturing the cells on the slide glass, and introducing the expression vectors into the cultured cells, thereby preparing a cell chip in which target protein-expressing cells are disposed in a lattice pattern so that a probe can act on the target protein expressed by each cell, has been also developed (J. Ziauddin & D. M. Sabatini, Nature 411: 107-110, 2001). In this method, the process of isolating and purifying proteins, or immobilizing and disposing proteins on a support can be omitted. However, for preparing such a cell chip, a special apparatus such as an arrayer or a dot blotter is needed to immobilize expression vectors on the spots one by one. And moreover, since expression vectors are immobilized on spots at regular intervals one by one, there are also problems in that the type of a target protein-expressing cell that can be immobilized and disposed on one support is limited, and that a number of target proteins cannot be examined with one cell chip. Furthermore, in the case of the foregoing cell chip, respective cells are specified by the type and the location of the expression vector immobilized on the spots on a slide glass, therefore, there are also problems in that it is inevitably limited to a solid phase screening, and that only the target protein expressed by a vector is a target for screening, and the difference in properties of cells per se cannot be a target for screening.

The invention of this application makes it an object to provide a new cell population which can be conveniently prepared without resort to a special apparatus and can be applied to both of a solid phase system and a liquid phase system in mass screening, and moreover, which is applicable to a screening for various properties of cells such as expression of a target protein.

In addition, the invention of this application makes it an object to provide a method of screening various properties of cells with the use of the foregoing cell population.

### Disclosure of Invention

The invention of this application provides a cell population with identification codes, which is a population of cells that can be distinguished from one another based on a difference in luminescent signals emitted by luminescent substances, wherein the difference in the luminescent signals is caused by either or both:

(a) 2 or more different luminescent properties; and
(b) 2 or more different luminescent sites.

In this cell population, a preferred aspect is that the luminescent substances of a part or all of the cells are fluorescent proteins, and/or that a part or all of the cells express a fusion protein of a fluorescent protein and a localization signal peptide.

Also in this cell population, preferred aspects are that each cell has a property different from the others, and further that the different property is expression of a different target protein.

Still furthermore, in this cell population, preferred aspects are that the cell is a eukaryotic cell, and that the eukaryotic cell is a mammalian cell.

Furthermore, another preferred aspect is that this cell population is immobilized and disposed in a minute area on a carrier.

This invention provides further a screening method for a cell property, which comprises contacting a probe with each cell of the cell population of any one of claims 4 to 8, and identifying the property of the cell binding to the probe with the use of the luminescent signal of the cell as an indicator.

In this screening method, preferred aspects are that the probe is a fusion protein of a probe protein and a fluorescent protein, and that the fluorescent protein has a luminescent property different from the luminescent properties that the cell population with identification codes has.

Further in this screening method, a preferred aspect is that the fusion protein probe is an in vitro transcription and translation product of a fusion gene of a probe protein gene and a fluorescent protein gene.

The foregoing aspects, terms or concept according to each invention will be defined in detail by referring to the description in the embodiments of the invention or Examples. In addition, various techniques to be used for carrying out this invention can be easily and surely carried out by those skilled in the art on the basis of known literatures and the like except for the techniques whose references are particularly specified. For example, the techniques of genetic engineering and molecular biology of this invention are described in Sambrook and Maniatis, in Molecular Cloning-A Laboratory Manual, Cold Spring Harbor Laboratory Press, New York, 1989: Ausubel, F. M. et al., Current Protocols in Molecular Biology, John Wiley & Sons, New York, N.Y, 1995, and the like.

**Brief Description of Drawings**

Fig. 1 is a diagram schematically showing a preparation procedure for a cell population with identification codes of this invention and a screening procedure with the use of this cell population.

Fig. 2 is a view showing the structure of fusion proteins which are introduced into the cell populations of this invention, respectively, as an Example. Figs. (a), (b), (c) and (d) show GPCL-XFP, MHIBDH-XFP, ACoABPL-XFP and ARH-XFP, respectively. Here, XFP represents any one of EGFP, EYFP and DsRED2. In addition, in the figure, TMD, MtLS and NLS represent a transmembrane domain, a mitochondria-localized signal and a Nucleus-localized signal, respectively.

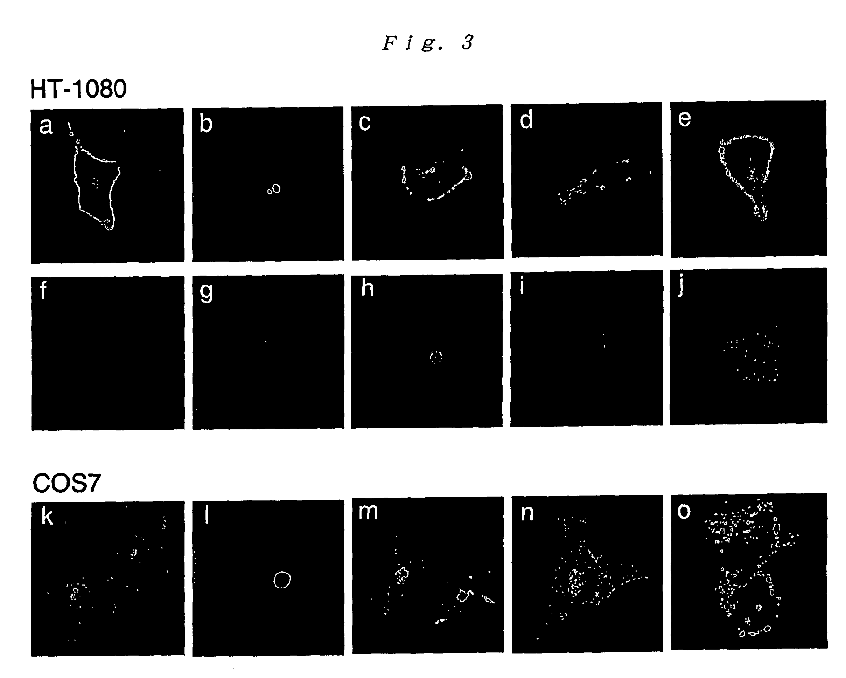

Fig. 3 shows confocal microscopic photographic images of a cell population with identification codes of this invention. The images show cells expressing the following combination: (a) GPCL-EGFP/ ARH-DsRed2, (b) GPCL-DsRed2/ ARH-EYFP, (c) GPCL-EYFP/ ARH-DsRed2, (d) GPCL-DsRed2/ MHIBDH-EGFP, (e) GPCL-EYFP/ MHIBDH-DsRed2, (f) ACoABPL-EGFP/ ARH-DsRed2, (g) ACoABPL-EYFP/ ARH-DsRed2, (h) ACoABPL-DsRed2/ ARH-EGFP, (i) GPCL-DsRed2/ ACoABPL-EGFP, (j) MHIBDH-DsRed2/ / ACoABPL-EGFP, (k) GPCL-EGFP/ ARH-DsRed2, (l) GPCL-DsRed2/ / ARH-EYFP, (m) GPCL-EYFP/ ARH-DsRed2, (n) GPCL-EGFP/ MHIBDH-DsRed2, and (o) MHIBDH-EGFP/ ARH-DsRed2. As the cell, HT-1080 cells were used for (a) to (j), and COS7 cells were used for (k) to (l).

Fig. 4 shows confocal microscopic photographic images of a protein chip consisting of cells expressing five types of fusion proteins (GPCL-EGFP, MHIBDH-EGFP, MHIBDH-DsRed2, ACoABPL-DsRed2, ARH-EYFP). Figs. (a) and (b) show a differential interference image and a fluorescence image, respectively. The unit of the scale is μm.

Fig. 5 is a diagram schematically showing a method of screening a cell chip containing cells expressing target proteins with the use of a fluorescent protein fusion probe prepared by in vitro transcription and translation.

Fig. 6 is a view showing the structure of the fusion proteins used in an Example. Figs. (a), (b) and (c) show NpwBP(P2)-EGFP, GPCL-Npw38 and GPCL-Npw38-DsRed2, respectively.

Fig. 7 shows the fluorescence spectra of the fluorescent protein fusion probe prepared by in vitro transcription and translation.

Fig. 8 shows confocal microscopic photographic images of fluorescence emission when a fluorescent protein fusion probe was bound to a target protein on a cell chip, and (a) shows green fluorescence in the case of using a cell chip containing a cell expressing GPCL-Npw38, (b) shows the superimposition of (a) and a differential interference image, (c) and (d) show green fluorescence and red fluorescence, respectively in the case of using a cell chip containing a cell expressing GPCL-Npw38-DsRed2, and (e) shows the superimposition of both images and a differential interference image.

**Best Mode for Carrying Out the Invention**

A cell population with identification codes of this invention is a population of cells that can be distinguished from one another based on the difference in luminescent signals emitted by luminescent substances, and is characterized in that the difference in the luminescent signals is caused by either or both:

(a) 2 or more different luminescent properties; and/or
(b) 2 or more different luminescent sites.

The term "different luminescent properties" means that one property can be distinguished from the other properties by observation with a microscope. Examples of such a property include color, the level of brightness and darkness and the like. In addition, the form of luminescence may be either fluorescence or phosphorescence.

With regard to "luminescent substance", a known natural luminescent substance or a chemically synthesized substance, for example, a fluorescent dye compound, a fluorescent protein, a fluorescent semiconductor (quantum dot) or the like can be used. As the fluorescent dye compound, fluorescein isothiocyanate (FITC), tetramethylrhodamine isothiocyanate (TRITC) or the like, as the fluorescent protein, green fluorescent protein (GFP) derived from luminescent jellyfish, its variant EGFP, EYFP (yellow fluorescence), ECFP (blue fluorescence), DsRed1 (red fluorescence), DsRed2, the green fluorescent protein hrGFP derived from Renilla, or the like, and as the fluorescent semiconductor, a variety of quantum dots with a different luminescent color can be used. In this invention, all the cells constituting one cell population may have the same type of luminescent substance as an identification code (for example, all the cells have fluorescent proteins with different colors as an identification code), or a part of the cells may have a fluorescent dye compound as an identification code, and other cells may have a fluorescent protein with a color different from that of the fluorescent dye compound as an identification code. In this invention, a preferred embodiment is that a particular part or all of the cells have a fluorescent protein as an identification code.

The term "different luminescent sites" means that the luminescent sites of cells are different. The sites of cell may be any as long as the luminescent sites are the localized sites capable of being specified by a microscopic observation. For example, organelles (mitochondrion, peroxisome, endosome and the like), nucleus, nuclear structures (nucleolus, spliceosome and the like), cytoplasmic structures (microtubule, actinfilament, intermediate filament and the like), inner membranes (endoplasmic reticulum, Golgi body and the like), cell membrane and the like can be exemplified.

With regard to combinations of "different luminescent properties" with "different luminescent sites" as described above (hereinafter referred to as "luminescent signal pattern" in some cases), the number of these combinations is represented by the following formula in the case where there are n (n is a natural number of 1 or more) types of luminescent sites and m (m is a natural number of 1 or more) types of luminescent properties for each luminescent site.

$$\sum_{i=1}^{N} {}_nC_1 \times m^i$$

For example, in the case of using 3 types of luminescent substances with a different luminescent property (for example, color) and setting luminescent sites to be 1 to 4 sites, total of 255 types of luminescent signal patterns are theoretically obtained in the following combinations.

In the case of 1 site: ${}_4C_1 \times 3 = 12$ types
In the case of 2 sites: ${}_4C_2 \times 3^2 = 54$ types
In the case of 3 sites: ${}_4C_3 \times 3^3 = 108$ types
In the case of 4 sites: ${}_4C_4 \times 3^4 = 81$ types

Furthermore, in the case of the combination of 4 colors and 4 sites, it brings to total of the luminescent signal patterns to 624 types.

In the case of 1 site: ${}_4C_1 \times 4 = 16$ types
In the case of 2 sites: ${}_4C_2 \times 4^2 = 96$ types
In the case of 3 sites: ${}_4C_3 \times 4^3 = 256$ types
In the case of 4 sites: ${}_4C_4 \times 4^4 = 256$ types

In order to localize a luminescent substance in a specific site of a cell and to allow it to emit light, a luminescent substance is bound to a specific site of a cell through an "intracellular localization inducer". As the intracellular localization inducer, a localization signal peptide, an antibody, a peptide containing a protein binding motif, a natural substance, a synthetic substance or the like can be used. The bond between a luminescent substance and an intracellular localization inducer may be any bond form such as a covalent bond, an ionic bond or a hydrophobic bond; however, a covalent bond, which is hard to break, is preferred. The bond between them may be mediated by a carrier (a protein, a synthetic polymer, an organic compound, an inorganic substance or the like).

Incorporation of a luminescent substance and/or an intracellular localization inducer into a cell can be carried out by appropriately combining methods such as incorporation through endocytosis, incorporation with the use of a transport carrier such as a transfection reagent, binding to the cell surface, intracellular production by gene expression. For example, if a gene encoding an intracellular localization inducer (for example, an antibody specifically binding to a fluorescent protein) is expressed in a specific site of a cell, and the fluorescent protein is introduced into this cell through endocytosis or the like, a luminescent protein can be localized in a specific site in a cell. Incidentally, in this invention, a cell population in which a luminescent substance is localized by a method of gene expression (intracellular expression of a fusion protein fusing a fluorescent protein with a localization signal peptide) is considered to be a preferred embodiment. In addition, a cell expressing a fusion protein fusing a fluorescent protein with a localization signal peptide may be all the cells constituting a cell population or may be a part of the cells. In other words, in this invention, as long as each cell constituting a cell population can be distinguished from other cells, cells having various luminescent substances as the identification code may be mixed. Such a mixture of cells can be carried out by selecting appropriate cells according to the type of cell or the type of cell characteristic or target protein, or the like.

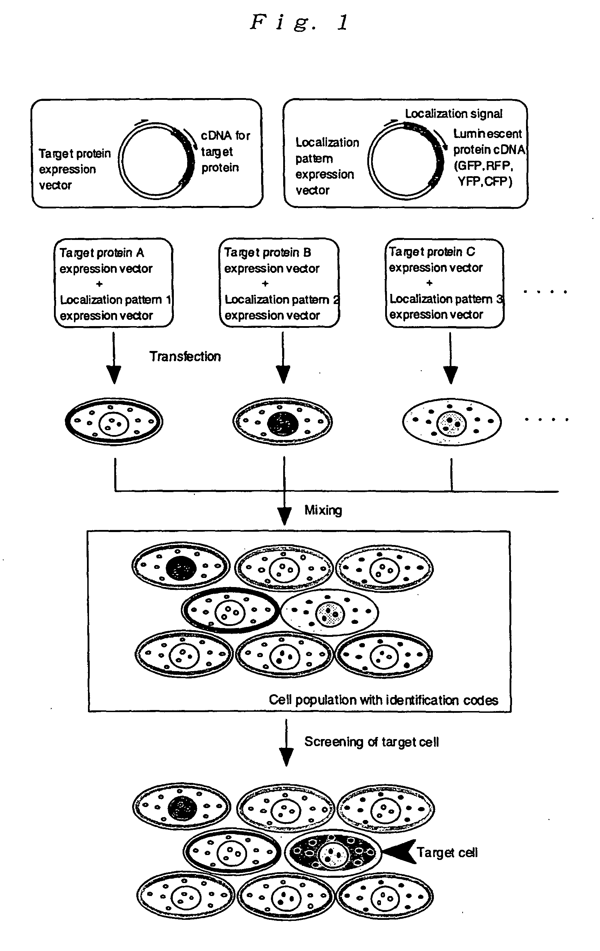

Fig. 1 shows a method of preparing a cell population expressing a fusion protein fusing a fluorescent protein with a localization signal peptide. That is, by fusing a localization signal peptide to the N-terminus or C-terminus of plural types of fluorescent proteins, a fluorescent protein expression vector with a localization signal is prepared. By introducing such expression vectors into cells in various combinations, and by coexpressing them, respectively, a desired luminescent localization pattern can be obtained. Examples of the fluorescent protein include a green fluorescent protein, a red fluorescent protein, a yellow fluorescent protein, a blue fluorescent protein and the like. As the localization signal peptide, a localization signal peptide causing localization in an organelle (mitochondrion, peroxisome, endosome or the like), the nucleus, a nuclear structure (nucleolus, spliceosome or the like), a cytoplasmic structure (microtubule, actinfilament, intermediate filament or the like), an inner membrane (endoplasmic reticulum, Golgi body or the like), cell membrane or the like is used. Incidentally, "localization signal peptide" may be a full-length protein with a localization signal, or a part of a protein containing a localization signal, or further, a peptide composed of an amino acid sequence constituting a localization signal.

As the expression vector, for example, in the case of using a eukaryotic cell as the subject, as long as it is an expression vector for a eukaryotic cell having a promoter, a splicing region, a poly (A) addition site and the like, it can be any vector, whether it be for example a plasmid vector or a virus vector, and pKA1, pCDM8, pSVK3, pMSG, pSVL, pBK-CMV, pBK-RSV, an EBV vector, pRS, pYES2 are examples. An expression vector is prepared by cloning a cDNA encoding a fluorescent protein and a DNA fragment encoding a localization signal in such a vector. In order to introduce an expression vector into a eukaryotic cell, a known method such as electroporation, the calcium phosphate method, the liposome method, the DEAE dextran method can be used. In addition, a fluorescent protein with a localization signal can be also expressed in a eukaryotic cell by a method according to a gene therapy method (ex vivo method) with the use of, for example, a hollow nanoparticle exhibiting a biological recognition molecule, a retrovirus, a lentivirus, an adenovirus, an adeno-associated virus or the like.

Incidentally, luminescent proteins showing different localization patterns have been frequently expressed in a cell by fusing a localization signal to a fluorescent protein (for example, CLONTECHniques April, 2000), however, so far, there has been no idea that by providing individual cells with different luminescent localization patterns, individual cells of a cell population are distinguished.

"Cells" constituting a cell population of this invention may be either prokaryotic cells or eukaryotic cells; however, eukaryotic cells are preferred because plural localization sites can be utilized, whereby more types of luminescent localization patterns can be obtained. As the eukaryotic cells, for example, cultured mammalian cells such as monkey kidney cells (COS7), Chinese hamster ovary cells (CHO) and various human tumor cell lines, budding yeast, fission yeast, silkworm cells, Xenopus laevis egg cells are examples. Alternatively, they may be primary cultured cells isolated from an animal. In addition, if mixed culture is possible, two or more types of eukaryotic cells derived from different species or different tissues may be used. Furthermore, they may be either floating cells or adherent cells; however, in the case where a cell population is used after immobilizing it on a support, adherent cells are preferred.

A first embodiment in a cell population of this invention is a population of cells whose luminescent signal patterns are different and other properties are the same. Such a cell population can be used for various screenings or assay methods by providing individual cells with a "different property" with the subsequent treatment.

A second embodiment in a cell population of this invention is a population of cells whose luminescent signal patterns are different, and which in addition have different properties. The term "different properties" in this case means to provide cells with a new genotype by introducing a foreign gene, to change the property by subjecting to a physical or a chemical treatment, or the like. Or it also means that, for example, because the species, organs or tissues from which cells are derived are different, the genotypes or phenotypes are individually different. For example, by introducing different protein expression vectors into a population of cells of the same type but whose respective luminescent signal patterns are different, a library of cells having different expressed target proteins can be constructed. Thus, the type of the target protein expressed by the cell reacting with a specific probe or the like can be immediately specified by the luminescent localization pattern of the cell (see Fig. 1). Or, for example, by a physical treatment with radiation or the like, or a chemical treatment with a carcinogenetic substance or the like, a cell population containing malignantly transformed cells derived from various tissues and the like can be obtained. Further, the type of the cell reacting with a specific probe (for example, a tumor cell-specific antibody or the like) can be immediately specified by the luminescent signal of the cell.

With regard to such a cell population having different properties, first, a cell population having different properties is prepared, and then different luminescent signal patterns are provided to the respective cells of this cell population. In addition, in the case where different properties are provided by introducing foreign gene expression vectors, and different luminescent signal patterns are provided by introducing expression vectors of fluorescent proteins with localization signals, introduction of two vectors is carried out at the same time, and the introduced genes may be coexpressed.

Incidentally, with regard to a "target protein" to be expressed by introduction of a foreign gene expression vector, any proteins derived from any biological species including human can be targeted. Its function may be either known or unknown. The amino acid sequence of the target protein or the DNA sequence encoding it may be unknown; however, it is preferred that they be known. The amino acid sequence may be either a sequence derived from a naturally occurring protein or an artificially designed sequence. Furthermore, this target protein may be either a polypeptide or an oligopeptide composed of a part of consecutive sequence of the amino acid sequence of a natural protein.

With regard to the cell population with identification codes of this invention, as described above, one cell can be distinguished from other cells because the luminescent signal pattern of each cell is different. Therefore, for example, if a property (for example, a target protein) of a cell is correlated with a luminescent signal pattern, it can be immediately determined what property or target protein the cell reacting with a specific probe expresses.

Also, the cell population with identification codes of this invention can be used by immobilizing respective cells on a support or in a floating state, according to the desired screening method or assay method. For example, these cells are suspended, and various reactions are carried out in a floating state, and the ones in which a reaction occurs can be selected from these. For example, by suspending 1,000 types of cells in a solution of volume in the order of μl or nl, an assay on an extremely small scale is possible. When a cell having a specific property is thus screened, that property of the cell can be immediately determined from the luminescent localization pattern found by observing the cell under a fluorescence microscope.

On the other hand, in the case where the one binding to a probe is screened from among the cells expressing target proteins, it is also favorable that a cell population be used while immobilized on a support (hereinafter, a cell population immobilized on a support is referred to as "cell chip" in some cases). This cell chip can be prepared by immobilizing and disposing a mixed culture of a cell population whose luminescent signal patterns are different and which has different properties in a minute area on a support at a high density. It is preferred that a population of eukaryotic cells expressing different target proteins, respectively, be used as a cell population. In this case, with regard to the "mixed culture of eukaryotic cells" immobilized and disposed on a support, plural types of eukaryotic cells each of which expresses a different target protein may be mixed in equal amounts (for example, one cell for each) and cultured, or the cells cultured and used of one or more specific types may be more or less numerous than other types. With regard to preparation of a mixed culture of eukaryotic cells, either a method of mixing cells after respective cells are cultured separately, or a method of culturing cells which have been mixed in advance can be employed. However, the former is preferred because the mixing ratio can be accurately controlled. In this case, cells cultured separately are detached from the incubator by a protease treatment or the like, each suspension containing a predetermined number of cells is prepared, and respective cell suspensions are fully mixed so as to suspend each cell uniformly. Thereafter, the cells are inoculated on the support of a cell chip, and culture is further continued. At this time, by controlling the number of inoculated cells, or by selecting the type of cells, a chip with a high cell density of a maximum number of 5,000 cells per 1 mm$^2$ can be obtained.

With regard to a support for culturing and immobilizing mixed cells, its material may be any as long as it can adhere to cultured cells and is transparent so as to enable microscope observation. For example, a slide glass or a culture container made of plastic can be used. In addition, a support whose cell adhesive ability at the surface is heightened by a coating treatment with a protein such as collagen or laminin, or by a chemical treatment, is also used.

In the cell chip prepared by the above method, plural types of eukaryotic cells in which the target proteins expressed by the respective cells can be specified based on the difference in the expression patterns are immobilized and disposed randomly in a minute area on a support at a high density. The target protein expressed by each cell disposed randomly can be identified based on the difference in the respective expression patterns as described above. Such a cell chip can be used for a screening by reacting it with a probe immediately after immobilizing eukaryotic cells on a support; or the cells may be immobilized with paraformaldehyde or the like. Furthermore, a cell chip can be stored in a freezer until use.

A screening method of this invention is characterized by bringing a probe in contact with each cell of a cell population with identification codes and identifying the property of the cell binding to the probe with the use of the luminescent signal of the cell as an indicator. The method can be carried out in a liquid phase system with a floating cell population as the subject or in a solid phase system with the foregoing cell chip. However, in the case where the subject is the binding between a target protein and a probe, it is preferred that the method be carried out in a solid phase system with a cell chip. In this screening method, since a cell chip on which cells are immobilized and disposed at a high density is used, the area for a screening becomes smaller, whereby the necessary amount of a probe can be a very small amount, on the order of μl's. For example, in the case of using a culture slide with a well of 0.4 cm$^2$ area, a screening of maximum 200,000 cells can be carried out by using 20 μl of a probe.

With regard to the binding between a target protein and a probe, in the case where the probe is a known substance or a known protein, an unknown target protein is identified as the protein binding to this. On the other hand, in the case where the target protein is known but it is not known whether some new substance (for example, a lead compound for developing a medicinal agent or the like) or proteins bind to the target protein, these substances may be used as probes.

In the case where the probe is a protein, this "probe protein" is a protein or a peptide for screening a desired protein from plural target candidate proteins according to specific binding ability to a target protein. With regard to the probe protein, any proteins derived from any biological species including human can be targeted. Its function may be either known or unknown. The amino acid sequence of a probe protein and the DNA sequence encoding it may be unknown, however, it is preferred that they be known. The amino acid sequence may be either a sequence derived from a naturally occurring protein or an artificially designed sequence. Furthermore, this probe protein may be a polypeptide or an oligopeptide consecutive portion of the amino acid sequence of a natural protein.

In addition, a probe is labeled with an enzyme, a radioisotope, a fluorescent dye or the like. The enzyme is not particularly limited as long as it meets the conditions that the metabolic turnover rate is high, it is stable even if it is bound to a probe candidate substance, it stains a substrate specifically and the like. And, for example, peroxidase, β-galactosidase, alkaline phosphatase, glucose oxidase, acetylcholinesterase, glucose-6-phosphate dehydrogenase, malate dehydrogenase or the like can be also used. The binding between these enzymes and probe candidate substances can be achieved by a known method with the use of a crosslinking agent such as a maleimide compound. As the substrate, a known substance can be used according to the type of an enzyme to be used. For example, in the case of using peroxidase as the enzyme, 3,3',5,5'-tetramethylbenzidine, and in the case of using alkaline phosphatase, paranitrophenol or the like can be used. As the radioisotope, the one used for a common RIA or the like such as $^{125}$I, $^3$H can be used. As the fluorescent dye, other than the one used in a common fluorescence method including fluorescein isothiocyanate (FITC), tetramethylrhodamine isothiocyanate (TRITC) and the like, a fluorescent protein such as green fluorescent protein can be used. However, in the case where a luminescent substance such as a fluorescent dye or a fluorescent protein is labeled, it is preferred that a luminescent substance having a luminescent property which is not contained in the cell population with identification codes be used as a label.

Still furthermore, the probe can be labeled as a fusion protein probe fusing a probe protein with a fluorescent protein. The fusion protein probe can be prepared by expressing a fusion gene fusing a probe protein gene (for example, cDNA or the like encoding the probe protein) with a fluorescent protein gene (cDNA) in an appropriate host-vector system. Or it can be also obtained as an in vitro transcription and translation product of a fusion gene.

In the case where a fluorescent protein fusion probe is prepared as an in vitro transcription and translation product, a fusion polynucleotide is recombined with an expression vector having an RNA polymerase promoter, and it was added to an in vitro transcription and translation system such as a rabbit reticulocyte lysate, a wheat germ extract, an E. coli lysate or the like containing an RNA polymerase corresponding to the promoter, whereby a fluorescent protein fusion probe can be prepared in vitro. In vitro transcription and in vitro translation may be carried out separately. As the RNA polymerase promoter, T7, T3, SP6 and the like can be exemplified. As a vector containing such an RNA polymerase promoter, pKA1, pCDM8, pT3/T7 18, pT7/3 19 pBluescript II, a pIVEX system and the like can be exemplified.

In the case where a fluorescent protein fusion probe is prepared with the use of a microorganism such as E. coli, an expression vector is prepared by recombining a fusion polynucleotide with an expression vector which has an origin, a promoter, a ribosome binding site, a DNA cloning site, a terminator and the like and is replicable in the microorganism, a host cell is transformed with this expression vector, and the obtained transformant is cultured, whereby a fusion protein encoded by this fusion polynucleotide can be produced in a microorganism in a large amount. As the expression vector for E. coli, a pUC system, pBluescript II, a pET expression system, a pGEX expression system and the like are examples.

In the case where a fluorescent protein fusion probe is prepared with the use of a eukaryotic cell, a fusion polynucleotide is recombined with an expression vector for a eukaryotic cell having a promoter, a splicing region, a poly (A) addition site and the like, and it is introduced into a eukaryotic cell, whereby a fluorescent protein fusion probe can be produced in a eukaryotic cell. As the expression vector, pKA1, pCDM8, pSVK3, pMSG, pSVL, pBK-CMV, pBK-RSV, an EBV vector, pRS, pYES2 and the like can be exemplified. As the eukaryotic cell, a cultured mammalian cell such as monkey kidney cell (COS7) or Chinese hamster ovary cell (CHO), budding yeast, fission yeast, a silkworm cell, a Xenopus laevis egg cell and the like are generally used, however, it may be any eukaryotic cell as long as it can express a fluorescent protein fusion probe.

After a fluorescent protein fusion probe is transcribed and translated in vitro or expressed in a prokaryotic cell or a eukaryotic cell, the obtained cell lysate can be used directly as a probe. In the case where a desired fluorescent protein fusion probe is isolated and purified from the culture, known separation procedures can be carried out in combination. For example, a treatment with a denaturant such as urea or a surfactant, sonication, enzymatic digestion, a salt precipitation or a solvent precipitation method, dialysis, centrifugation, ultrafiltration, gel filtration, SDS-PAGE, isoelectric focusing, ion exchange chromatography, hydrophobic chromatography, affinity chromatography, reverse phase chromatography and the like can be used.

In the screening method of this invention, after contacting labeled probes with cells, detection is carried out by a known method corresponding to signals from the probes with the labels. In the case of using an enzyme as a label, a substrate which can develop color due to decomposition by an enzymatic action is added, and the amount of decomposed substrate is optically measured. In the case of using a radioisotope, the radiation emitted from a radioisotope is detected by autoradiography or the like. In addition, in the case of using a fluorescent dye or a fluorescent protein, the fluorescence amount may be measured with a measuring device connected to a fluorescence microscope. However, in the case of a target protein localized in a cell, a method of using a fluorescently labeled probe and detecting the binding by a microscopic observation is preferred. If the binding is observed at the site where a target protein is localized by an observation under a microscope, it is highly possible that the binding is the binding of the probe to the target protein. Incidentally, in the case of a target protein being an intracellular protein or a cell in which a target protein is localized in a cell and using a probe which cannot penetrate cell membrane, after a cell is treated with a surfactant or an organic solvent to make the cell membrane permeable, it is reacted with the probe.

## Examples

Hereunder, the invention of this application will be explained in more detail and specifically by showing Examples; however, the invention of this application is not intended to be limited to these Examples. Incidentally, basic procedures and enzymatic reactions related to DNA recombination followed the literature ("Molecular Cloning. A laboratory manual", Cold Spring Harbor Laboratory, 1989). With regard to restriction enzymes and various modification enzymes, the ones manufactured by Takara, Shuzo Co. Ltd. were used unless otherwise particularly stated. Composition of a buffer solution for each enzymatic reaction and a reaction condition followed the attached instruction.

## Example 1

### Preparation of cell population with identification codes

(1) Preparation of expression vector

(1-1) Fluorescent protein expression vector

Fluorescent protein expression vectors, pKA1-EGFP-N1, pKA1-EYFP-N1 and pKA1-DsRed2-N1, were prepared by inserting an EcoRI-NotI fragment containing cDNA of a fluorescent protein (EGFP, EYFP or DsRed2), which had been prepared from pEGFP-N1, pEYFP-N1 and pDsRed2-N1 respectively (all from Clontech), into the EcoR1-NotI site of pKA1 (Kato et al., Gene 150: 243-250, 1994).

(1-2) Membrane-localized fluorescent protein expression vector

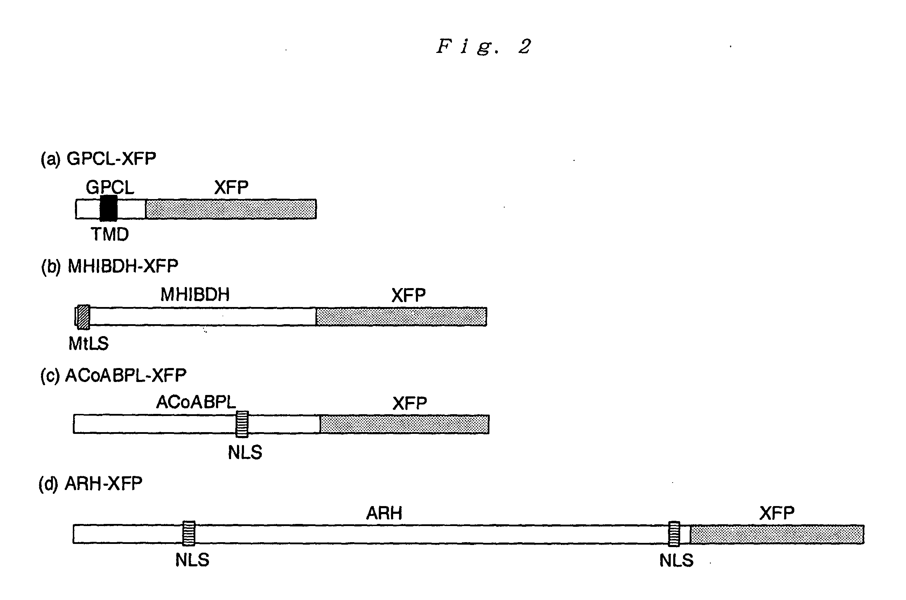

A PCR product was prepared by using a T7 primer and a primer to which a SmaI site had been added at the downstream of the stop codon with the use of pHP10524 (described in WO 00/00506 PCT Gazette) harboring cDNA encoding a human glycophorin C-like protein (GPCL) as a template. After this PCR product was digested with EcoRI and SmaI, it was inserted into the EcoRI-SmaI cleavage site of the respective fluorescent protein expression vectors prepared in the foregoing (1-1), pKA1-EGFP-N1, pKA1-EYFP-N1and pKA1-DsRed2-N1, whereby membrane-localized fluorescent protein expression vectors, pKA1-GPCL-EGFP, pKA1-GPCL-EYFP and pKA1-GPCL-DsRed2, were prepared. A schematic view of the fusion proteins, GPCL-EGFP, GPCL-EYFP and GPCL-DsRed2, is shown in Fig. 2(a).

(1-3) Mitochondria-localized fluorescent protein expression vector

A PCR product was prepared by using a T7 primer and a primer to which a BamHI site had been added at the downstream of the stop codon with the use of pHP00698 (described in JP-A-2001-037482) harboring cDNA encoding a human mitochondrial 3-hydroxyisobutyrate dehydrogenase (MHIBDH) as a template. After this PCR product was digested with EcoRI and BamHI, it was inserted into the EcoRI-BamHI cleavage site of the respective fluorescent protein expression vectors, pEGFP-N1, pEYFP-N1 and pDsRed2-N1, whereby mitochondria-localized fluorescent protein expression vectors, pMHIBDH-EGFP, pMHIBDH-EYFP and pMHIBDH-DsRed2, were prepared. A schematic view of the fusion proteins, MHIBDH-EGFP, MHIBDH-EYFP and MHIBDH-DsRed2, is shown in Fig. 2(b).

(1-4) Nucleus-localized fluorescent protein expression vector

A PCR product was prepared by using a T7 primer and a primer to which a KpnI site had been added at the downstream of the stop codon with the use of pHP01124 (described in JP-A-2001-333781) harboring cDNA encoding a human acyl-CoA binding protein-like protein (ACoABPL) as a template. After this PCR product was digested with EcoRI and KpnI, it was inserted into the EcoRI-KpnI cleavage site of the respective fluorescent protein expression vectors prepared in the foregoing (1-1), pKA1-EGFP-N1, pKA1-EYFP-N1 and pKA1-DsRed2-N1, whereby nucleus-localized fluorescent protein expression vectors, pKA1-ACoABPL-EGFP, pKA1-ACoABPL-EYFP and pKA1- ACoABPL-DsRed2, were prepared. A schematic view of the fusion proteins, ACoABPL-EGFP, ACoABPL-EYFP and ACoABPL -DsRed2, is shown in Fig. 2(c).

(1-5) Nucleolus-localized fluorescent protein expression vector

A PCR product was prepared by using a T7 primer and a primer to which a SmaI site had been added at the downstream of the stop codon with the use of pHP02644 (described in JP-A-2001-218584) harboring cDNA encoding a human ATP- dependent RNA helicase (ARH) as a template. After this PCR product was digested with EcoRI and SmaI, it was inserted into the EcoRI-SmaI cleavage site of the respective fluorescent protein expression vectors prepared in the foregoing (1-1), pKA1-EGFP-N1, pKA1-EYFP-N1 and pKA1-DsRed2-N1, whereby nucleolus-localized fluorescent protein expression vectors, pKA1-ARH-EGFP, pKA1-ARH-EYFP and pKA1-ARH- DsRed2, were prepared. A schematic view of the fusion proteins, ARH-EGFP, ARH-EYFP and ARH-DsRed2, is shown in Fig. 2(d).

(2) Provision of identification codes to culture cells

(2-1) Cultured cells

A human fibrosarcoma cell line, HT-1080 and monkey kidney cells, COS7, were cultured in Dulbecco's modified Eagle's medium (DMEM) containing 10% fetal bovine serum (FBS) at 37°C in the presence of 5% $CO_2$. HT-1080 cells ($2 \times 10^5$ cells) were inoculated into a 6-well multidish (Nunc) and cultured at 37°C in the presence of 5% $CO_2$ for 22 hours. After the medium was removed, the surface of the cells was washed with a phosphate buffer solution (PBS), and further 1.5 ml of DMEM containing 10% FBS was added.

(2-2) Introduction of expression vector into cells

DNA complexes were prepared by coupling the expression vectors prepared in Example 1 (1-2) to (1-5), singly or in combination with 2 types, with one type of cDNA expression vector selected from a human full-length cDNA bank (Seishi Kato, BIO INDUSTRY 11: 760-770, 1994) which was different with respect to each combination, adding 1 μl (corresponding to 1.5 μg) of the respective solutions to 100 μl of serum-free DMEM, mixing the solution with 10 μl of PolyFect™ transfection reagent (Qiagen) and incubating the solution at room temperature for 10 minutes. The cultured HT-1080 cells or COS7 cells prepared in the foregoing (2-1) were washed once with PBS, and 1.5 ml of DMEM containing 10% FBS was added. The previously prepared DNA complexes, to which 600 μl of DMEM containing 10% FBS had been added, were added to these cells, and cultured at 37°C in the presence of 5% $CO_2$ for 22 hours.

(3) Observation of luminescent localization pattern

After the cultured cells were washed with PBS, the cells were immobilized with PBS containing 4% paraformaldehyde at room temperature for 15 minutes. When this was observed with a confocal fluorescence microscope (Bio-Rad, MRC1024ES), a cell population showing various luminescent patterns according to the introduced fusion proteins was obtained. In this Example, since localization was set to one site and two sites and three fluorescent proteins were used, a cell population showing 12 types and 54 types, respectively, and the total 66 types of fluorescent patters was obtained. Fig. 3 shows a part of the luminescent patterns in the case of localization in two sites. The photographs show the examples of HT-1080 cell population expressing fluorescent proteins of different colors in the following sites: cell membrane and nucleoli ((a) to (c)); cell membrane and mitochondria ((d) and (e)); nucleus and nucleoli ((f) to (h)); cell membrane and nucleus (i); and mitochondria and nucleus (j). Though the types of cells are different, a basic luminescent pattern does not change; however, there is a case where the localization is slightly different. For example, in the case where cells are localized in cell membrane by using COS7 cells, the whole cell membrane is luminescent and accumulation also in the endoplasmic reticulum around the nucleus is observed (see (k) to (n)). Even if there is such a difference, by confirming the pattern when a protein has been expressed singly in advance, it does not hinder identification of the localized site. The respective cells express different target proteins; therefore, by performing a variety of screening with the use of this cell population, and observing the luminescent pattern of cells sorted out as a result of the screening, it can be immediately identified which target protein the cell expresses since the target protein expression vector introduced with the luminescent pattern expression vector has been known.

**Example 2**

**Preparation of cell chip**

(1) Cell chip A

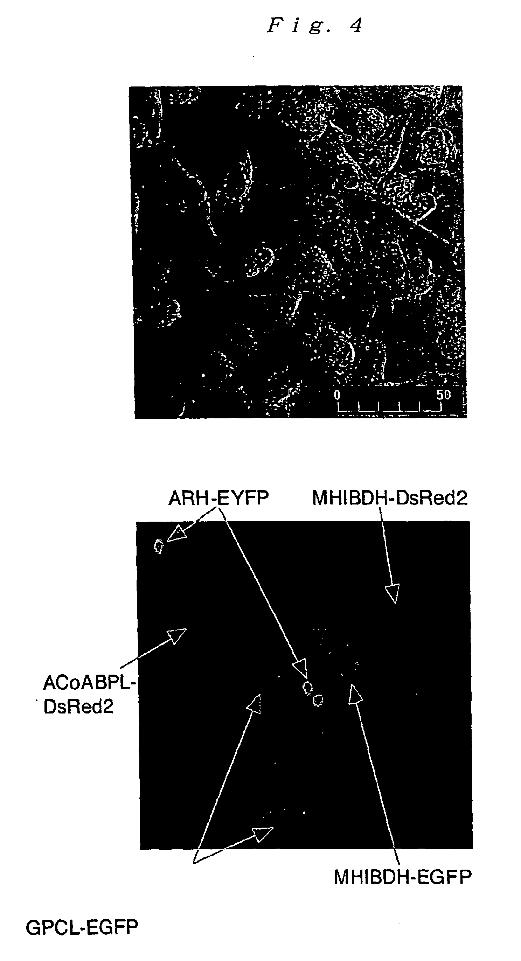

Five types of fusion protein-expressing cells prepared in Example 1 (2-2) were reacted with 1 ml of 0.05% Trypsin-EDTA solution at 37°C for 5 minutes, respectively, and detached from the culture substrate. After the cells were recovered by adding 2 ml of DMEM containing 10% FBS, these five types of fusion protein-expressing cells were prepared to have a density of 2 x $10^5$ cells/ml, and mixed uniformly. A 1 ml portion of this cell mixture suspension was plated on a collagen I culture slide (Falcon), and further cultured at 37°C in the presence of 5% $CO_2$ for 32 hours. After the cells were washed with PBS, the cells were immobilized with PBS containing 4% paraformaldehyde at room temperature for 15 minutes, whereby a cell chip A was prepared.
This cell chip A was observed with a confocal fluorescence microscope (Bio-Rad, MRC1024ES), and the fluorescence derived from the GFP fusion protein expressed by each cell on the chip was measured. The results are shown in Fig. 4. The cell expressing five types of fusion proteins can be observed in the visual field of the microscope. The cell density at this time was about 1,300 cells/$mm^2$.

(2) Cell chip B

In the same manner as above, HT-1080 cells into which pKA1-EGFP-N1 prepared in Example 1 (1-1) and pKA1 (control vector) had been introduced, respectively, were prepared. Each cell was mixed at a ratio of 1:100, and 100 µl of this cell mixture suspension was inoculated into a 16-well chamber slide (Nunc), and a cell chip B was prepared in the same method as in Example 4.

(3) Screening with the use of antibody

After the cell chip B prepared in the foregoing (2) was washed with PBS, it was treated with 0.1% Triton X-100. This was reacted with 20 µl of an anti-GFP antibody in 10% Block Ace (Dainihon Pharmaceutical) for 90 minutes, washed with PBS, and reacted with a rhodamine-conjugated secondary antibody in 10% Block Ace for 40 minutes. As a result of observing the distribution of red fluorescence derived from the antibody with a confocal fluorescence microscope, the cells emitting red fluorescence were observed at a ratio of 10 cells/$mm^2$. The cells emitting red fluorescence also showed the original green fluorescence of EGFP.
From the above results, it was confirmed that by using the cell chip of this invention, the presence or absence of a target protein at a ratio of one every 100 cells can be investigated with the use of a trace amount of probe (antibody).

**Example 3**

**Screening for protein-protein interaction**

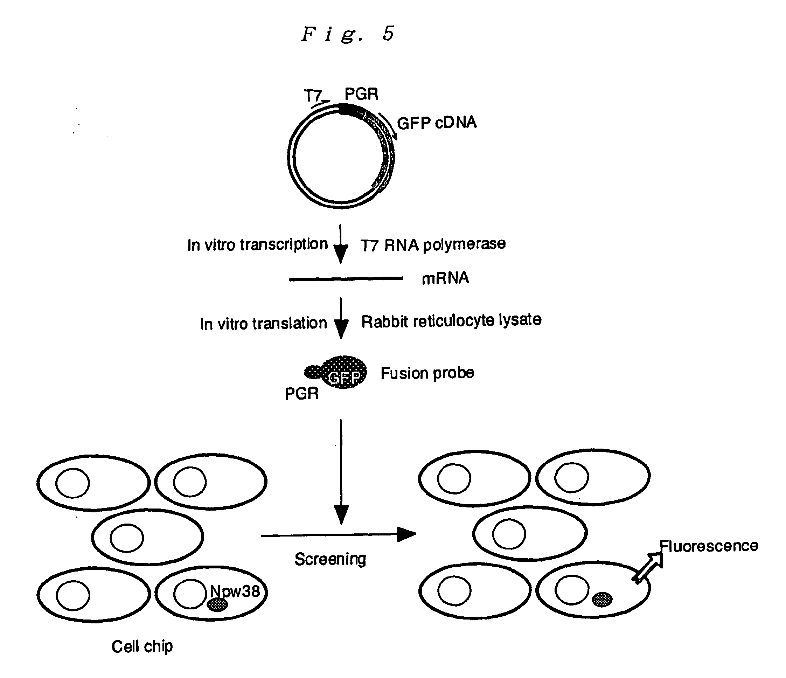

An example is here described of a screening by selecting the binding between Npw38 and NpwBP, which are nuclear proteins, as the model interaction, and using NpwBP as the probe protein and Npw38 as the target protein. Namely, it is known that the WW domain of Npw38 binds to the PGR motif of NpwBP (A. Komuro et al., J. Biol. Chem. 274: 36513-36519, 1999). Therefore, a peptide containing the PGR motif of NpwBP was selected as the probe protein. The product of fusing this peptide to a fluorescent protein was used as a fluorescent protein fusion probe to screen a cell chip containing the cells expressing membrane-localized Npw38 (see Fig. 5).

(1) Preparation of expression vector

(1-1) Fluorescent protein expression vector

Fluorescent protein expression vectors, pKA1-EGFP-N1and pKA1-DsRed2-N1, were prepared by inserting an EcoRI-NotI fragment containing cDNA of a fluorescent protein (EGFP or DsRed2) which had been prepared from each of pEGFP-N1and pDsRed2-N1(both from Clontech) into the EcoRI-NotI site of pKA1 (Kato et al., Gene 150: 243-250, 1994).

(1-2) Fluorescent protein fusion probe expression vector

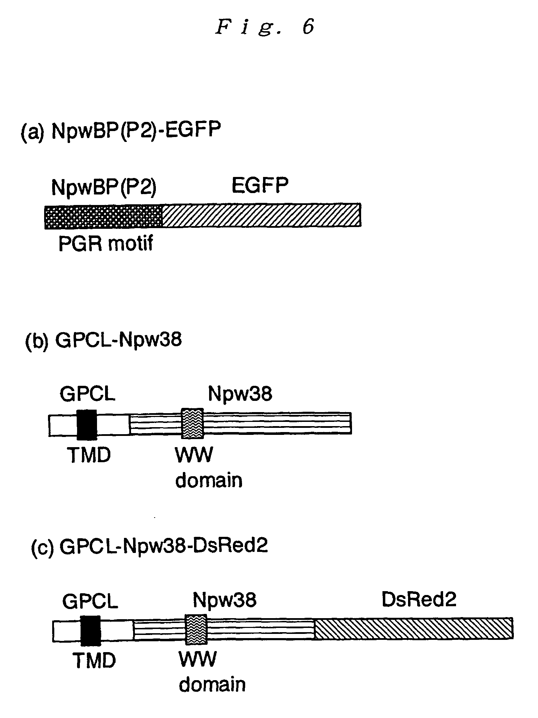

A vector, pKA1-NpwBP (P2)-GFP (described in JP-A- 2001-327296) expressing a fusion protein fusing PGR motif peptide of NpwBP with GFP (see Fig. 6 (a)) was used as a fluorescent protein fusion probe expression vector. This vector has a T7 RNA polymerase promoter at the upstream of the cDNA, therefore, if T7 RNA polymerase is made to act on it, in vitro transcription is initiated and mRNA encoding the fusion protein can be synthesized (see Fig. 5).

(1-3) Preparation of GPCL fusion protein expression vector

A PCR product was prepared by using a T7 primer and a primer to which a SmaI site had been added at the downstream of the stop codon, with pHP10524 (described in WO 00/00506 PCT Gazette) harboring cDNA encoding a human glycophorin C-like protein (GPCL) as a template. After this PCR product was digested with EcoRI and SmaI, it was inserted into the EcoRI-SmaI cleavage site of the respective fluorescent protein expression vectors pKA1-EGFP-N1 and pKA1-DsRed2-N1 prepared in the foregoing (1-1), whereby membrane-localized fluorescent protein expression vectors, pKA1-GPCL-EGFP and pKA1-GPCL-DsRed2, were prepared.

(1-4) Preparation of GPCL-Npw38 fusion protein expression vector

A PCR product was prepared by using a T3 primer and a primer to which a SmaI site had been added at the upstream of the start codon with pKA1-Npw38 (A. Komuro et al., Nucl. Acids Res. 27: 1957-1965, 1999) as a template. After this PCR product was digested with SmaI and NotI, it was inserted into the SmaI-NotI cleavage site of the membrane-localized fluorescent protein expression vector, pKA1-GPCL-EGFP, prepared in the foregoing (1-3), whereby GPCL-Npw38 fusion protein expression vector, pKA1-GPCL-Npw38, was prepared. A schematic view of the fusion protein, GPCL-Npw38, is shown in Fig. 6(b).

(1-5) Preparation of GPCL-Npw38-DsRed2 fusion protein expression vector

A PCR product was prepared by using a primer to which a SmaI site had been added at the upstream of the start codon and a primer containing a SmaI site which is present in Npw38 with pKA1-Npw38 as a template. After this PCR product was digested with XmaI, it was inserted into the XmaI cleavage site of the membrane-localized fluorescent protein expression vector, pKA1-GPCL-DsRed2, prepared in the foregoing (1-3), whereby GPCL-Npw38-DsRed2 fusion protein expression vector, pKA1-GPCL-Npw38-DsRed2, was prepared. A schematic view of the fusion protein, GPCL-Npw38-DsRed2, is shown in Fig. 6(c).

(2) Preparation of cell chip

(2-1) Cultured cells

A human fibrosarcoma cell line, HT-1080 was cultured in Dulbecco's modified Eagle's medium (DMEM) containing 10% fetal bovine serum (FBS) at 37°C in the presence of 5% $CO_2$. HT-1080 cells (2 x $10^5$ cells) were inoculated into a 6-well multidish (Nunc) and cultured at 37°C in the presence of 5% $CO_2$ for 22 hours. After the medium was removed, the surface of the cells was washed with a phosphate buffer solution (PBS), and further 1.5 ml of DMEM containing 10% FBS was added.

(2-2) Introduction of expression vector into cells

DNA complexes were prepared by adding 1 μl (corresponding to 1.5 μg) of the solutions of expression vectors prepared in Example 3 (1-2) and (1-3) (pKA1-GPCL-Npw38, pKA1-GPCL-Npw38-DsRed2 or pHP10524 as a control vector that expresses only GPCL) to 100 μl of serum-free DMEM, mixing the solution with 10 μl of PolyFect™ transfection reagent (Qiagen) and incubating the solution for 10 minutes at room temperature. The cultured HT-1080 cells prepared in the foregoing (2-1) were washed once with PBS, and 1.5 ml of DMEM containing 10% FBS was added. The previously prepared DNA complexes, to which 600 μl of DMEM containing 10% FBS had been added, were added to these cells, and cultured at 37°C in the presence of 5% $CO_2$ for 22 hours.

(2-3) Immobilization of fusion protein-expressing cells on support

The fusion protein-expressing cells prepared in the foregoing (2-2) were reacted with 1 ml of 0.05% Trypsin-EDTA solution at 37°C for 5 minutes, respectively, and detached from the culture dish. After the cells were recovered by adding 2 ml of DMEM containing 10% FBS, these fusion protein-expressing cells were prepared at a density of 2 x $10^5$ cells/ml, and uniformly mixed with the cells expressing only GPCL. A 1 ml portion of this cell mixture suspension was plated on a collagen I culture slide (Falcon), and further cultured at 37°C in the presence of 5% $CO_2$ for 40 hours. After the cells were washed with PBS, the cells were immobilized with PBS containing 4% paraformaldehyde at room temperature for 15 minutes, whereby a cell chip was prepared.

(3) Preparation of fluorescent protein fusion probe

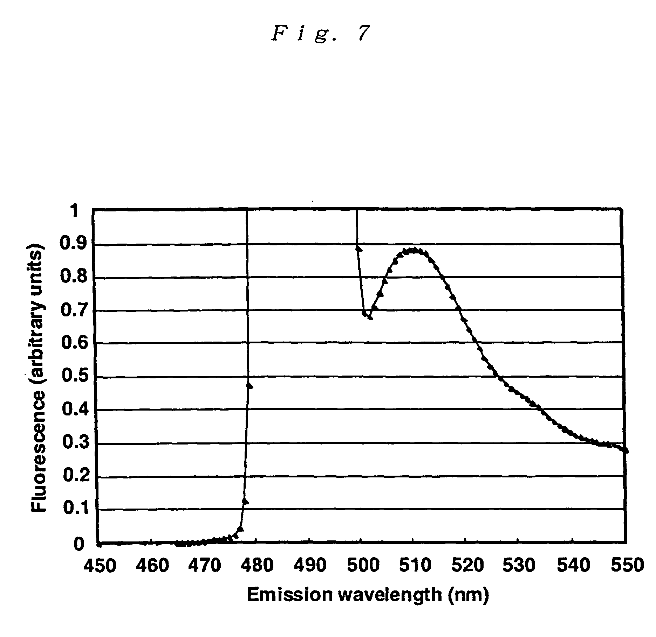

By adding 1 μg of pKA1-NpwBP(P2)-GFP described in the foregoing (1-2) to the total amount of 50 μl of a solution containing 40 μl of $T_NT^R$ Quick Master Mix and 1 μl of 1 mM methionine included in an in vitro transcription/ translation reaction kit (Promega Co.), the reaction was carried out at 30°C for 12 hours. This reaction solution was directly used as a probe for screening. A portion of the reaction solution was taken out and diluted 200-fold with PBS, and the fluorescence spectra (excitation light: 488 nm) were measured with a fluorescence spectrophotometer. The results are shown in Fig. 7. The fluorescence emission with a maximum at 510 nm was observed, and it was demonstrated that the fusion protein of the translation product, NpwBP(P2)-GFP, functions as GFP.

(4) Screening with the use of fluorescent protein fusion probe

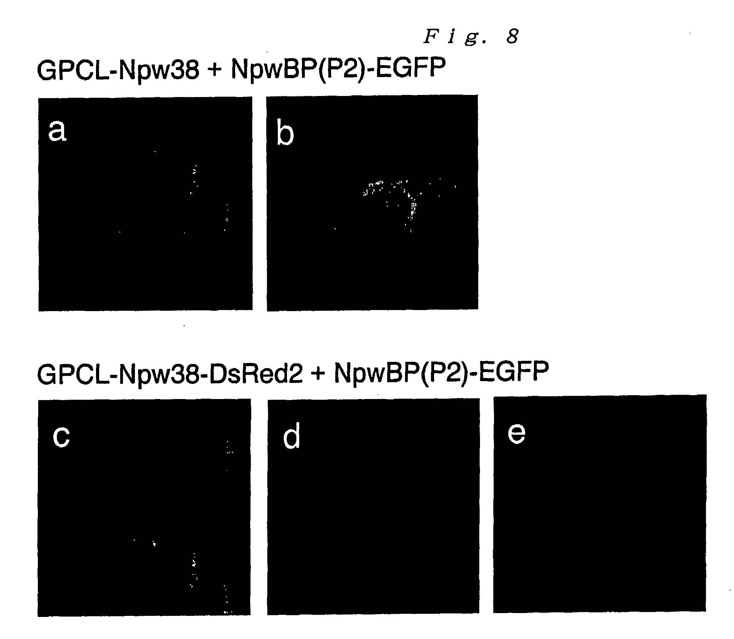

After the cell chip containing cells expressing GPCL-Npw38 prepared in the foregoing (2) was washed with PBS, it was treated with 0.1% Triton X-100 on the ice for 15 minutes. To the cells on this chip, 20 μl of the fluorescent protein fusion probe, NpwBP(P2)-GFP, prepared in the foregoing (3), was added, enclosed, and reacted at 4°C for 20 hours. After it was washed three times with PBS containing 0.05% Tween 20 and enclosed, it was observed with a confocal fluorescence microscope (Bio-Rad, MRC1024ES). As a result, the cells emitting green fluorescence in the endoplasmic reticulum around the nucleus were observed (Fig. 8(a) and (b)). In order to confirm that this site that emits fluorescence coincides with the localization site of GPCL-Npw38, the same experiment was carried out by using the cell chip containing the cells expressing GPCL-Npw38-DsRed2 in which further a red fluorescent protein DsRed2 had been fused to GPCL-Npw38. As a result, in the same manner as the case of GPCL-Npw38, the cells emitting green fluorescence were observed in the endoplasmic reticulum around the nucleus (Fig. 8 (c)). Furthermore, this site emitting green fluorescence agreed with the site emitting red fluorescence that indicated the localized site of GPCL-Npw38-DsRed2 (Fig. 8(d) and (e)), therefore, it was confirmed that the fluorescent protein fusion probe, NpwBP(P2)-GFP, is bound to GPCL-Npw38-DsRed2. Meanwhile, the binding of the probe was not observed in the cells expressing only GPCL, therefore, it was demonstrated that this binding is mediated by Npw38.

**Industrial Applicability**

As described in detail above, by this application, a novel cell population which can be conveniently prepared without a resort to a special apparatus and can be applied to either a solid phase system or a liquid phase system in mass screening, and moreover, which can be used in a screening for various properties of cells such as the expression of a target protein is provided.

**Claims**

1. A cell population with identification codes, which is a population of cells that can be distinguished from one another based on a difference in luminescent signals emitted by luminescent substances, wherein the difference in the luminescent signals is caused by either or both:

   (a) 2 or more different luminescent properties; and
   (b) 2 or more different luminescent sites.

2. The cell population with identification codes of claim 1, wherein the luminescent substances of a part or all of the cells are fluorescent proteins.

3. The cell population with identification codes of claim 1 or 2, wherein a part or all of the cells express a fusion protein of a fluorescent protein and a localization signal peptide.

4. The cell population with identification codes of claim 1, wherein each cell has a different property.

5. The cell population with identification codes of claim 4, wherein the different property is expression of a different target protein.

6. The cell population with identification codes of claim 5, wherein the cell is a eukaryotic cell.

7. The cell population with identification codes of claim 6, wherein the eukaryotic cell is a mammalian cell.

8. The cell population with identification codes of any one of claims 4 to 7, which is arranged and immobilized in a minute area on a carrier.

9. A screening method for a cell property, which comprises contacting a probe with each cell of the cell population of any one of claims 4 to 8, and identifying the property of the cell binding to the probe with the use of the luminescent signal of the cell as an indicator.

10. The screening method of claim 9, wherein the probe is a fusion protein of a probe protein and a fluorescent protein.

11. The screening method of claim 10, wherein the fluorescent protein has a luminescent property different from the luminescent properties that a cell population with identification codes has.

12. The screening method of claim 10 or 11, wherein the fusion protein probe is an in vitro transcription and translation product of a fusion gene of a probe protein gene and a fluorescent protein gene.

*F i g. 1*

(a) GPCL-XFP

(b) MHIBDH-XFP

(c) ACoABPL-XFP

(d) ARH-XFP

Fig. 3

*F i g .  4*

ARH-EYFP          MHIBDH-DsRed2

ACoABPL-
DsRed2

MHIBDH-EGFP

GPCL-EGFP

# Fig. 5

T7 PGR

GFP cDNA

In vitro transcription → T7 RNA polymerase

———————— mRNA

In vitro translation → Rabbit reticulocyte lysate

PGR GFP Fusion probe

Cell chip

Npw38

Screening

Fluorescence

EP 1 496 119 A1

# *F i g.  6*

### (a) NpwBP(P2)-EGFP

NpwBP(P2)　　　　　EGFP

PGR motif

### (b) GPCL-Npw38

GPCL　　　　　Npw38

TMD　　　　WW
domain

### (c) GPCL-Npw38-DsRed2

GPCL　　　Npw38　　　　　DsRed2

TMD　　　WW
domain

*F i g .  7*

*F i g. 8*

GPCL-Npw38 + NpwBP(P2)-EGFP

GPCL-Npw38-DsRed2 + NpwBP(P2)-EGFP

<table>
<tr><td colspan="2" style="text-align:center"><b>INTERNATIONAL SEARCH REPORT</b></td><td>International application No.<br>PCT/JP03/04180</td></tr>
</table>

| A. CLASSIFICATION OF SUBJECT MATTER |
|---|
| Int.Cl⁷ C12N15/09, 5/10, C12Q1/02, G01N33/50 |

According to International Patent Classification (IPC) or to both national classification and IPC

| B. FIELDS SEARCHED |
|---|
| Minimum documentation searched (classification system followed by classification symbols)<br>Int.Cl⁷ C12N15/00-15/90, 5/10, C12Q1/00-1/70, G01N21/75-21/83, 21/62-21/74 |

Documentation searched other than minimum documentation to the extent that such documents are included in the fields searched

Electronic data base consulted during the international search (name of data base and, where practicable, search terms used)
MEDLINE(STN), WPI/BIOSIS(DIALOG), JSTPlus(JOIS)

### C. DOCUMENTS CONSIDERED TO BE RELEVANT

| Category* | Citation of document, with indication, where appropriate, of the relevant passages | Relevant to claim No. |
|---|---|---|
| P,X<br>P,A | Fumie FUJIMORI et al., "Shikibetsu Code tsuki Saibo Chip", Seikagaku, August 2002, Vol.74, No.8, page 1095 | 1-8<br>9-12 |
| Y<br>A | JP 11-146798 A (Railway Technical Research Institute),<br>02 June, 1999 (02.06.99),<br>(Family: none) | 1-8<br>9-12 |
| Y<br>A | EP 121262 A2 (Becton, Dickinson and Co.),<br>10 August, 1984 (10.10.84),<br>& JP 59-184862 A | 1-8<br>9-12 |
| Y<br>A | JP 9-56384 A (Toray Industries, Inc.),<br>04 March, 1997 (04.03.97),<br>(Family: none) | 1-8<br>9-12 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>17 April, 2003 (17.04.03) | Date of mailing of the international search report<br>30 April, 2003 (30.04.03) |
|---|---|
| Name and mailing address of the ISA/<br>Japanese Patent Office | Authorized officer |
| Facsimile No. | Telephone No. |

Form PCT/ISA/210 (second sheet) (July 1998)

## INTERNATIONAL SEARCH REPORT

| International application No. |
| --- |
| PCT/JP03/04180 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y<br>A | WO 91/01305 A1  (University of Wales College of Medicine),<br>07 February, 1991 (07.02.91),<br>& EP 484369 A1          & US 5683888 A | 1-8<br>9-12 |
| Y<br>A | WO 00/17221 A1  (Duke University),<br>30 March, 2000 (30.03.00),<br>& EP 1115734 A1 | 1-8<br>9-12 |
| A | KOMURO A. et al., Association of Two Nuclear Proteins, Npw38 and NpwBP, via the Interaction between the WW Domain and a Novel Proline-rich Motif Containing Glycine and Arginine. J.Biol.Chem., 1999, Vol.274, No.51, pages 36513 to 36519 | 1-12 |

Form PCT/ISA/210 (continuation of second sheet) (July 1998)